Europäisches Patentamt

(19) European Patent Office

Office européen des brevets

(11) Publication number: **0 323 652 B1**

# (12) EUROPEAN PATENT SPECIFICATION

(45) Date of publication of patent specification:
**06.11.91 Bulletin 91/45**

(51) Int. Cl.⁵: **A61K 7/48, A61K 7/00**

(21) Application number: **88200013.6**

(22) Date of filing: **07.01.88**

(54) **Cosmetic skin treatment.**

(43) Date of publication of application:
**12.07.89 Bulletin 89/28**

(45) Publication of the grant of the patent:
**06.11.91 Bulletin 91/45**

(84) Designated Contracting States:
**AT BE CH DE ES FR GB GR IT LI LU NL SE**

(56) References cited:
**EP-A- 0 114 581**
**FR-M- 7 323**
**US-A- 4 752 472**

(73) Proprietor: **EXOVIR, INC.**
**111 Great Neck Road**
**Great Neck New York 11021 (US)**

(72) Inventor: **Kligman, Albert M.**
**637 Pine Street**
**Philadelphia Pennsylvania 19406 (US)**

(74) Representative: **Smulders, Theodorus A.H.J.,**
**Ir. et al**
**Vereenigde Octrooibureaux Nieuwe Parklaan**
**107**
**NL-2587 BP 's-Gravenhage (NL)**

## Description

This invention relates to the cosmetic treatment of facial skin and the like for the removal of materials from the skin surface and from the sebaceous follicles.

A technique known as "follicular biopsy", an extension of the non-invasive "surface biopsy", has been developed to extract the contents of sebaceous follicles so that the retrieved material can be examined histologically at the light or electron microscopic level, see the publication "The Follicular Biopsy" by O.H. Mills & A.M. Kligman, Dermatologica, 167, 57 (1983). The disclosures of this publication are herein incorporated and made part of this disclosure.

The follicles of human facial skin secrete a horny material (follicular horn) throughout the life of the individual. This material does not normally build up in the pores but is extruded and is removed by facial cleansing. At about age 50, and particularly in the skin of females, the follicles tend to dilate and fill up with horny material, which traps small vellus hairs. This debris distends the follicular orifices, giving them the appearance of blackheads. The surface of the skin becomes coarse and uneven. The lay term fo these horny-filled follicles is "clogged pores".

In addition to the horny material in the follicles, other retention products such as bacteria (P. acnes) fungi (P. ovale) and a mite (Demodex folliculorum) contribute to the follicular debris.

Certain cutaneous disorders, such as acne, keratosis pilaris, ichthyotic states and fungal infections involve abnormal and excessive keratinization. As does the age-related build up of follicular horn, these conditions also impart a course and inflamed look to the skin.

In all of the above described conditions, removal of the horny material and other debris from the follicles returns the follicles to their normal, healthy state and imparts a clear, smooth look to the skin surface. However, no simple method has heretofore existed for the removal of deep follicular horn and debris.

It would thus be desirable to provide a simple method for the complete removal of follicular horn and associated debris from the skin follicles.

Since removal of follicular horn and debris from the skin returns the skin to a clear, smooth state, such removal would be of a substantial interest to the cosmetics industry.

It is an object of the present invention to provide a cosmetic treatment for the removal of follicular horn and debris from the skin.

The invention is directed to the cosmetic treatment of facial skin and the like for the removal of materials from the surface and the sebaceous follicles of human skin. The cosmetic treatment comprises applying a layer of liquid polymerizable adhesive to the skin, allowing polymerization of the polymerizable adhesive, and removing or stripping the resulting layer of polymerized adhesive from the skin. In this treatment, the materials on the surface of the skin and the sebaceous follicles in contact with the applied polymerizable adhesive become attached to and removed with the stripped layer of polymerized adhesive.

Specifically, the cosmetic treatment involves the removal of surface material from the skin and from the sebaceous follicles of the skin by applying a thin layer or coating of a liquid polymerizable cyanoacrylate adhesive to the skin to be treated, followed by contacting the cyanoacrylate layer with an adhesive tape to adhere the tape to the cyanoacrylate layer and thus removing the polymerized cyanoacrylate layer from the skin by lifting or peeling or stripping away the tape away from the skin taking with it the polymerized cyanoacrylate layer and attached skin surface and follicular materials.

This cosmetic treatment for the removal of follicular horn and debris from the skin pores comprises applying a liquid polymerizable alpha-cyanoacrylate to the skin to coat the skin and to enter and invade the affected pores so as to remove the unwanted material from the skin along with the resulting polymerized adhesive. The method not only completely cleans the skin and the pores of the skin but also imparts a healthy, clear and smooth look to the skin.

The liquid polymerizable adhesives of this invention are preferably cyanoacrylate-based adhesives such as liquid alpha-cyanoacrylate. Other liquid adhesives which produce similar results may be employed. Presently preferred are the alpha-alkyl cyanoacrylates such as alpha-methyl cyanoacrylate. The cyanoacrylate adhesive is presently used in the commercially available form, e.g. Krazy Glue adhesive, although agents which influence or increase or decrease the polymerization time, increase invasion of the applied cyanoacrylate adhesive into the pores and follicles and fragrances and surfactants and the like may be added.

The liquid cyanoacrylate adhesive is brushed or coated or applied onto the skin, after the skin has been prepared by washing and drying, using any convenient applicator or technique. The liquid adhesive quickly invades the follicles where it surrounds and infiltrates the horn and other debris within the follicles.

Before the adhesive completely polymerizes on the skin (usually within about three minutes) a flexible, pliable patch or backing or strip of adhesive tape is applied, sufficient to cover the treated area and pressed thereto so as to establish firm contact of the tape with the applied cyanoacrylate coating.

The backing or tape usually carries its own adhesive layer, and may be composed of paper, plastic, cloth or any flexible, pliable material. Presently preferred materials are the hypoallergenic adhesive coated tapes, such as Blenderm™ manufactured by 3M Company and Dermacil™, Dermilite™ and Dermiclear™ manufactured by Johnson & Johnson. After applying the backing or tape to the treated area, about three minute elapses before the cyanoacrylate layer or coating is fully polymerized. When polymerized, an edge of the backing or tape is grasped, such as with the fingers or with any suitable instrument, and the backing pulled away from the skin.

Contents of the follicles remain attached to the backing due to the infiltration of the now-polymerized cyanoacrylate layer. A little discomfort is sometimes experienced when the backing is removed and when such discomfort is experienced, as may be evidence by some reddening of the treated skin, a hyper-emollient cream is applied to soothe any skin irritation.

While a variety of hyper-emollient creams may be employed, a presently preferred composition is as follows :

Hyper Emollient Cream
(Face and Body)

| | |
|---|---|
| Ultimata (Penreco White Petrolatum) | 6.5% |
| Frost (Penreco White Petrolatum) | 5.0% |
| Amercol-Cab | 30.0% |
| Cetyl Alcohol (95.0%) | 2.5% |
| Cyclochem-GMS-165 | 6.0% |
| Polawax | 3.0% |
| Myristyl Myristate (Croda) | 2.5% |
| Procetyl AWS | 2.5% |
| Crodamol PMP | 2.5% |
| Methyl Paraben | 0.2% |
| Propyl Paraben | 0.1% |
| Water | 30.7% |

Presently preferred treatment regimens are once every two to four weeks, or less frequent, such as once every 2-4 months, although certain conditions may require more frequent treatment for optimum effect. Treatment regimens of once per week for six weeks have been followed with no adverse effects noted.

Example

A female patient with follicular horn buildup presented for treatment. The skin was washed with warm water and mild soap, and patted dry. Alpha methyl cyanoacrylate at ambient temperature was brushed onto an affected area of facial skin, leaving a thin layer of the unpolymerized adhesive. Within one minute, and before polymerization of the applied cyanoacrylate layer was completed, Blenderm™ adhesive tape was applied to cover the cyanoacrylate layer and pressed thereto. After approximately three minutes, the edge of the tape was grasped and the tape was pulled sharply away from the skin surface. On visual observation, material could be seen attached to the tape forming a pattern that reproduced the pattern of pores of the patient's skin.

This technique was repeated until all affected areas were treated. Treatment overlapping into unaffected skin areas frequently occurred but was not detrimental to the patient.

Following this cosmetic treatment, a hyperemollient soothing cream was rubbed onto the treated skin by the patient when it was noted that the skin was slightly red immediately after treatment.

Although the patient had presented with coarse, uneven skin that appeared to be dotted with blackheads, within four hours after the treatment the skin was clear and smooth, and the post-treatment redness had disappeared. Treatments were continued once per week for six weeks and no ill effects were noted. The treated skin looked healthy, clean and relatively smooth.

Although it is preferred to apply the tape to the adhesive coated skin before the adhesive has substantially completely polymerized, other suitable techniques as to when to apply the tape may be employed. The tape may be applied to the adhesive coating after the adhesive coating has completely polymerized. Also additional one or more coatings of the cyanoacrylate adhesive may be applied to the first applied adhesive coating, either before or after the first applied adhesive coating has polymerized and the tape then applied, also before or after the last applied adhesive coated has polymerized or otherwise reacted to form a coherent film or coating on the skin. In the practice of this invention the tape is applied to the adhesive coating such that when the tape is removed it takes with it and strips away the cyanoacrylate adhesive coating the skin, together with the skin surface debris and the follicular debris now fixed to the cyanoacrylate adhesive coating on the tape.

## Claims

1. A method for the cosmetic treatment of facial skin and the like for the removal of materials from the surface and the sebaceous follicles of human skin which comprises applying a coating or film of liquid polymerizable adhesive to said skin, allowing the polymerization of said coating of polymerizable adhesive, and removing the coating of polymerized adhesive from the skin, the materials from the surface of the skin and the sebaceous follicles being attached to and removed with the layer of polymerized adhesive.

2. A method of claim 1, wherein the materials include bacteria, fungi, mites, and follicular horn.

3. A method of claim 1, wherein the adhesive is a liquid polymerizable cyanoacrylate.

4. A method of claim 3 wherein said coating of polymerizable adhesive comprises alpha methyl cyanoacrylate.

5. A method of claim 1, wherein said coating of polymerizable adhesive is applied at a substantially uniform thickness on the skin by brushing.

6. A method of claim 1, wherein said coating of polymerized adhesive is removed by contacting the layer with a second adhesive on a pliable backing or tape and lifting or peeling away said backing or tape from the skin.

7. A method of claim 6, wherein the backing or tape is cloth.

8. A method of claim 6, wherein the backing or tape is paper.

9. A method of claim 6, wherein the backing or tape is plastic such as polyester.

10. A cosmetic method for the removal of surface material from the skin and from the sebaceous follicles of the skin which comprises applying a thin, substantially uniform coating or film of a liquid polymerizable cyanoacrylate adhesive to the skin, contacting the cyanoacrylate layer with an adhesive tape, said tape adhering to said cyanoacrylate coating, and removing the polymerized cyanoacrylate coating or film from the skin by lifting or peeling away said tape away from the skin.

11. A method of claim 10, wherein the materials include bacteria, fungi, mites and follicular horn.

12. A method of claim 10, wherein the cyanoacrylate is alpha methyl cyanoacrylate.

13. A method of claim 10, wherein the tape is a plastic tape.

14. A method of claim 10, wherein the tape is a cloth tape.

15. A method of claim 10, wherein the tape is a paper tape.

16. A method of claim 10, wherein said coating or film of polymerizable adhesive is applied in an amount sufficient to uniformly wet and coat the skin surface to be treated.

17. A method according to claim 10, wherein another coating of polymerizable adhesive is applied to the coating of cyanoacrylate adhesive before contact with said adhesive tape.

18. A method according to claim 17, wherein said other coating of polymerizable adhesive is applied before polymerization of the first applied coating of liquid polymerizable cyanoacrylate adhesive.

19. A method according to claim 17, wherein said other coating of polymerizable adhesive is applied after polymerization of the first applied coating of liquid polymerizable cyanoacrylate adhesive.

20. A method according to claim 17, wherein said adhesive tape is applied to said other coating of polymerizable adhesive before said other coating has polymerized.

21. A method according to claim 17, wherein said adhesive tape is applied to said other coating of polymerizable adhesive after said other coating has polymerized.

## Patentansprüche

1. Ein Verfahren zur Kosmetischen Behandlung der Gesichtshaut und Ähnlichem zur Entfernung von Oberflächenmaterial und von den Talgfollikeln der menschlichen Haut, was das Aufbringen einer Schicht oder eines Films aus einem flüssigen, polymerisierbaren Haftmittel auf die besagte Haut beinhaltet, wodurch die Polyme-

4

risierung der besagten Schicht oder des polymerisierbaren Haftmittels ermöglicht wird, sowie die Entfernung der Schicht aus polymerisiertem Haftmittel von der Haut, des Materials von der Hautoberfläche und der Talgfollikel, die sich an die Schicht aus polymerisiertem Haftmittel geheftet haben und mit dieser entfernt werden.

2. Ein Verfahren entsprechend Anspruch 1, wobei das Oberflächenmaterial Bakterien, Schimmel, Milben und follikulares Horngewebe umfaßt.

3. Ein Verfahren entsprechend Anspruch 1, wobei das Haftmittel ein flüssiges, polymerisierbares Cyanoacrylat ist.

4. Ein Verfahren entsprechend Anspruch 3, wobei die besagte Schicht aus polymerisierbarem Haftmittel Alphamethyldyanoacrylat enthält.

5. Ein Verfahren entsprechend Anspruch 1, wobei die besagte Schicht aus polymerisierbarem Haftmittel in einer im wesentlichen gleichförmigen Stärke durch Bürsten auf die Haut aufgebracht wird.

6. Ein Verfahren entsprechend Anspruch 1, wobei die besagte Schicht aus polymerisierbarem Haftmittel dadurch entfernt wird, daß die Schicht mit einem zweiten Haftmittel auf einem biegsamen Träger oder auf einem Band in Berührung gebracht und der besagte Träger oder das Band von der Haut abgehoben oder abgeschält wird.

7. Ein Verfahren entsprechend Anspruch 6, wobei der Träger oder das Band ein Tuch ist.

8. Ein Verfahren entsprechend Anspruch 6, wobei der Träger oder das Band aus Papier besteht.

9. Ein Verfahren entsprechend Anspruch 6, wobei der Träger oder das Band aus Kunststoff, wie etwa Polyester, besteht.

10. Ein kosmetisches Verfahren zur Entfernung von Oberflächenmaterial von der Haut und von den Talgfollikeln der Haut, welches darin besteht, daß eine dunne, im wesentlichen gleichförmige Schicht oder ein ebensolcher Film aus flüssigem, polymerisierbarem Cyanoacrylathaftmittel auf die Haut aufgetragen wird, wobei die Cyanoacrylatschicht mit einem Klebeband in Betuhrung gebracht wird, sich das besagte Band mit der besagten Cyanoacrylatschicht verbindet und die polymerisierte Cyanoacrylatschicht oder der entsprechende Film durch Abheben oder Abschälen des besagten Bandes von der Haut entfernt wird.

11. Ein Verfahren entsprechend Anspruch 10, wobei das Oberflächenmaterial Bakterien, Schimmel, Milben und follikulares Horngewebe umfaßt.

12. Ein Verfahren entsprechend Anspruch 10, wobei das Cyanoacrylat Alphamethylcyanoacrylat ist.

13. Ein Verfahren entsprechend Anspruch 10, wobei das Band ein Kunststoffband ist.

14. Ein Verfahren entsprechend Anspruch 10, wobei das Band ein Stoffband ist.

15. Ein Verfahren entsprechend Anspruch 10, wobei das Band ein Papierband ist.

16. Ein Verfahren entsprechend Anspruch 10, wobei die besagte Schicht oder der besagte Film aus polymerisierbarem Haftmittel in ausreichender Menge aufgetragen wird, um die zu behandelnde Hautoberfläche gleichmäßig zu befeuchten und zu beschichten.

17. Ein Verfahren entsprechend Anspruch 10, wobei eine andere Schicht aus polymerisierbarem Haftmittel auf die Schicht aus Cyanoacrylathaftmittel aufgetragen wird, bevor die Berührung mit dem besagten Klebeband stattfindet.

18. Ein Verfahren entsprechend Anspruch 17, wobei die besagte andere Schicht aus polymerisierbarem Haftmittel vor der Polymerisierung der zuerst aufgetragenen Schicht aus flüssigem, polymerisierbarem Cyanoacrylathaftmittel aufgebracht wird.

19. Ein Verfahren entsprechend Anspruch 17, wobei die besagte andere Schicht aus polymerisierbarem Haftmittel nach der Polymerisierung der zuerst aufgetragenen Schicht aus flüssigem, polymerisierbarem Cyanoacrylathaftmittel aufgebracht wird.

20. Ein Verfahren entsprechend Anspruch 17, das besagte Klebeband auf die besagte andere Schicht aus polymerisierbarem Haftmittel aufgetragen wird, bevor die Polymerisierung der besagten anderen Schicht eingetreten ist.

21. Ein Verfahren entsprechend Anspruch 17, wobei das besagte Klebeband auf die besagte andere Schicht aus polymerisierbarem Haftmittel aufgebracht wird, nachdem sich die besagte andere Schicht polymerisiert hat.

## Revendications

1. Méthode de traitement cosmétique pour la peau du visage et analogue pour l'enlèvement de matières de la surface et des follicules sébacés de la peau humaine, qui comporte l'application d'un revêtement ou d'un film d'adhésif polymérisable liquide sur la dite peau, permettant la polymérisation du dit revêtement d'adhésif polymérisable, et l'enlèvement du revêtement d'adhésif polymérisé de la peau, des matières de la surface de la peau et des follicules cébacés fixées à, et enlevés avec la couche d'adhésif polymérisé.

2. Méthode selon la revendication 1, dans laquelle les matières comportent des bactéries, des champignons, des acariens et de la corne folliculaire.

3. Méthode selon la revendication 1, dans laquelle l'adhésif est un cyanoacrylate polymérisable liquide.

4. Méthode selon la revendication 3, dans laquelle le dit revêtement d'adhésif polymérisable comprend de l'alpha-méthyl cyanoacrylate.

5. Méthode selon la revendication 1, dans laquelle le dit revêtement d'adhésif polymérisable est appliqué selon une épaisseur essentiellement uniforme sur la peau par brossage.

6. Méthode selon la revendication 1, dans laquelle la dite couche d'adhésif polymérisé est enlevée en contactant la couche au moyen d'un second adhésif sur un support ou un ruban pliable et en soulevant ou en détachant le dit support ou ruban de la peau.

7. Méthode selon la revendication 6, dans laquelle le support ou le ruban est constitué par un tissu.

8. Méthode selon la revendication 6, dans laquelle le support ou le ruban est constitué par du papier.

9. Méthode selon la revendication 6, dans laquelle le support ou le ruban est constitué par du plastique, tel que du polyester.

10. Méthode cosmétique pour l'enlèvement des matières superficielles de la peau et des follicules sébacés de la peau comprenant l'application d'un revêtement ou d'un film fin, essentiellement uniforme, d'un adhésif constitué de cyanoacrylate polymérisable liquide sur la peau, le contact de la couche de cyanoacrylate par un ruban adhésif, le dit ruban adhérant à la couche de cyanoacrylate, et l'enlèvement du revêtement ou du film de cyanoacrylate polymérisé de la peau en soulevant ou en détachant le dit ruban de la peau.

11. Méthode selon la revendication 10, dans laquelle les matières comprennent des bactéries, des champignons, des acariens et de la corne folliculaire.

12. Méthode selon la revendication 10, dans laquelle le cyanoacrylate est de l'alpha-méthyl cyanoacrylate.

13. Méthode selon la revendication 10, dans laquelle le ruban est un ruban plastique.

14. Méthode selon la revendication 10, dans laquelle le ruban est un ruban en tissu.

15. Méthode selon la revendication 10, dans laquelle le ruban est un ruban en papier.

16. Méthode selon la revendication 10, dans laquelle le dit revêtement ou le dit film d'adhésif polymérisable est appliqué selon une quantité suffisante pour humidifier et revêtir uniformément la surface de peau à traiter.

17. Méthode selon la revendication 10, dans laquelle un autre revêtement d'adhésif polymérisable est appliqué sur le revêtement d'adhésif de cyanoacrylate avant le contact avec le dit ruban adhésif.

18. Méthode selon la revendication 17, dans laquelle le dit autre revêtement d'adhésif polymérisable est appliquée avant la polymérisation du premier revêtement appliqué d'adhésif de cyanoacrylate polymérisable liquide.

19. Méthode selon la revendication 17, dans laquelle le dit autre revêtement d'adhésif polymérisable est appliqué après la polymérisation du premier revêtement appliqué d'adhésif de cyanoacrylate polymérisable liquide.

20. Méthode selon la revendication 17, dans laquelle le ruban adhésif est appliqué sur le dit autre revêtement d'adhésif polymérisable avant la polymérisation de ce dernier.

21. Méthode selon la revendication 17, dans laquelle le dit ruban adhésif est appliqué sur le dit autre revêtement d'adhésif polymérisable après la polymérisation de ce dernier.